# EUROPÄISCHE PATENTSCHRIFT

(11) **EP 0 871 508 B1**
(45) Veröffentlichungstag und Bekanntmachung des Hinweises auf die Patenterteilung: **22.09.1999**
(21) Anmeldenummer: 96946229.0
(22) Anmeldetag: 23.12.1996
(51) Int. Cl.: A61M 5/44, A61M 1/36

(54) **ELEKTRISCHES SCHNELLAUFTAUGERÄT**
QUICK-THAWING ELECTRICAL APPARATUS
SYSTEME ELECTRIQUE DE DECONGELATION TRES RAPIDE

(30) Priorität: 27.12.1995 DE 19548826
(43) Veröffentlichungstag der Anmeldung: 21.10.1998
(73) Patentinhaber: Schmidt, Günther, 51429 Bergisch-Gladbach (DE)
(72) Erfinder: Schmidt, Günther, 51429 Bergisch-Gladbach (DE)
(74) Vertreter: Neumann, Andreas
(86) Internationale Anmeldenummer: DE9602508
(87) Internationale Veröffentlichungsnummer: WO9724152

(56) Entgegenhaltungen:
- EP-A- 0 282 958
- WO-A-88/07384
- CH-A- 648 486
- DE-A- 3 047 784
- US-A- 3 657 517

## Beschreibung

Die Erfindung betrifft ein elektrisches Schnellauftaugerät zum raschen Auftauen einer gefrorenen Flüssigkeit, beispielsweise einer Injektions- oder Infusionslösung, mit einem Übertragungsteller, einer Spann-Heizeinrichtung zur Aufnahme eines die gefrorene Flüssigkeit enthaltenden Behältnisses, insbesondere von einer Spritze oder einer Ampulle, welche eine elektrisch beheizbare Heizeinrichtung mit mindestens einer Heizfläche und mindestens einen die Temperatur an der Außenwand des Behältnisses messenden Temperaturmeßfühler umfaßt, sowie einer elektrischen Regeleinrichtung mit einem Temperaturregler mit Ansteuerschaltung, wobei der Temperaturmeßfühler und ein Sollwertgeber ausgangsseitig mit der Ansteuerschaltung verbunden sind, die Ansteuerschaltung ausgangsseitig mit einer Schalteinrichtung zur Steuerung der Heizeinrichtung verbunden ist, wobei die Schalteinrichtung in Abhängigkeit von Signalen des an dem Eingang der Ansteuerschaltung angeschlossenen Temperaturmeßfühlers und Sollwertgebers die Heizeinrichtung steuert, der Übertragungsteller an einen elektrischen Linearantrieb gekoppelt und im wesentlichen in einer Ebene bewegbar ist und auf dem Übertragungsteller die Spann-Heizeinrichtung koppelbar ist.

In der klinischen Praxis ist es üblich, pharmazeutische Wirkstoffe, die von der Industrie in Standardkonzentrationen und Standardvolumina geliefert werden, mit Trägerlösungen auf patientenspezifische Konzentration zu verdünnen und zu verabreichen. Da diese individuell angesetzten Präparate oft in langfristigen Therapien eingesetzt werden, wird bei der Herstellung der patientenspezifischen Wirkstoffmischung ein entsprechend großes Gesamtvolumen angesetzt. Die für die Einzelverabreichungen vorgesehenen Teilvolumina des Gesamtvolumens müssen deshalb in Spritzen oder anderen Behältnissen abgefüllt und anschließend gefroren aufgehoben werden. Nur so können die in der Regel thermisch labilen Wirkstoffe über die geforderten langen Zeiträume konserviert werden.

Ein Problem dieses eingeführten und weit verbreiteten herkömmlichen Konservierungsverfahrens ist es, daß für die Einzelproben jeweils eine relativ lange Auftauzeit vorzusehen ist. Dies ist vor allem auch insofern umständlich, als der Zeitpunkt der Verabreichung des nächsten Wirkstoffvolumens nicht exakt vorherbestimmt werden kann. Es wird für den einzelnen Patienten zwar ein Zeitplan für die Verabreichung der Wirkstoffvolumina erstellt; in der Regel wird dem Patienten jedoch zunächst eine Blutprobe entnommen, um erst nach deren Analyse zu entscheiden, ob und - falls ja - zu welchem genauen Zeitpunkt das nächste Wirkstoffteilvolumen appliziert werden kann. Diese Vorgehensweise führt daher oft zu Zeitplanverschiebungen und folglich zum Verlust der vorab aufgetauten Wirkstoffe.

Um unter anderem dieses Zeit- und Kostenproblem zu beseitigen, besteht das dringende Bedürfnis, ein Schnellauftaugerät bereitzustellen, welches die Wirkstoffteilvolumina rasch aufzutauen vermag.

Im Stand der Technik sind Mikrowellenauftaugeräte für gefrorene Flüssigkeiten bekannt, welche durch Mikrowellen bei Frequenzen zwischen 2,425 und 2,475 GHz erwärmt werden. Diese weisen jedoch den Nachteil auf, daß überwiegend die gefrorene Flüssigkeit aufgrund der Fokussierung der Strahlung in einem bestimmten Bereich der gefrorenen Flüssigkeit stark erwärmt wird, so daß eine gleichmäßige Erwärmung der gefrorenen Flüssigkeit nicht erfolgt. Die Erhitzung der gefrorenen Flüssigkeit nur in einem bestimmten Bereich kann zu einer Schädigung von Inhalts- oder Wirkstoffen der Flüssigkeit führen, so daß mit einer chemischen Modifikation der Inhalts- bzw. der Wirkstoffe zu rechnen ist. Insbesondere zeigt sich hierbei, daß das Auftauen von Infüsionslösungen oder Injektionslösungen in Spritzen oder Ampullen mittels herkömmlicher Mikrowellenauflaugeräten zu vermeiden ist, da sowohl eine chemische Modifikation der Arzneimittel als auch eine Denaturierung von Impfstoffen, Proteinen oder Seren in den Infusions- und Injektionslösungen nicht auszuschließen ist.

Zudem ist festzustellen, daß durch die Schädigung der medizinisch wirksamen Inhaltsstoffe der Injektions- und Infusionslösungen deren medizinische und therapeutische Wirksamkeit mit der Zunahme der Auftaudauer abnimmt. Hinzutretend zeigt es sich, daß die dadurch bedingte Abnahme der medizinischen Wirkung eine höhere Dosierung und damit eine höhere Konzentration der medizinisch wirksamen Inhaltsstoffe in den Infusions- und Injektionslösungen erforderlich macht, so daß ein kostenträchtigerer Mehrverbrauch sich einstellt.

Weiterhin ist auf die gesundheitsschädigende Wirkung durch die bisher verwendeten Verfahren zum schnellen Auftauen, wie Verfahren unter Verwendung von Mikrowelle, Hot-Spots-Bildung, heißem fließenden Wasser etc., beispielsweise modifizierter Wirkstoffe oder eingedrungener Mikroorganismen, wie Krankheitskeime usw., hinzuweisen, deren nachteilige Langzeiteffekte lediglich zum Teil aufgeklärt sind; jedoch jedenfalls zu hinreichenden Belastung des Organismusses während der Abbauphase im Körper des Verbrauchers bzw. Patienten führen.

Auch zeigt sich, daß durch die Verwendung von Drehtellern in Mikrowellenauftaugeräten keine gleichmäßige Erwärmung gefrorener Injektionslösungen besonders in länglichen Behältnissen, wie sie vorzugsweise im medizinischen Bereich Verwendung finden, beispielsweise in Spritzen oder Ampullen, möglich ist, da trotz des zeitweise durch das Drehen des Drehtellers hervorgerufenen Verlassens des Fokussierungsbereichs die örtliche Erhitzung der gefrorenen Flüssigkeit derart groß ist, daß die o.g. Schädigungen der in den Infusions- und Injektionslösungen befindlichen Arzneimittel eintreten und kein schonendes Auftauen stattfindet.

Ebenso die zum Auftauen von mit gefrorenen Injektionslösungen gefüllten Spritzen übliche Handhabung, nämlich z. B. Spritzen unter warmem fließendem Wasser zu erwärmen, kann zu einer Kontamination der Flüssigkeit durch Mikroorganismen führen, welche von außen in dem Spalt zwischen dem Spritzenkolben und dem Spritzenkörper -von dem Wasserstrahl eingedrückt- einzudringen vermögen

Die klassischen Verfahren des Auftauens, z. B. Erhitzen der Wirkstoffvolumina, Verwendung von Mikrowellenschränken, Überspülen der Spritzen unter fließendem warmen Wasser etc., schließen sich folglich aus Gründen der thermischen Labilität der zu verabreichenden Wirkstoffe sowie der zwingend erforderlichen Sterilität des Mediums aus. Aus diesen Gründen ist man angewiesen, die Probe bei Zimmertemperatur mit dem großen Nachteil der langen Auftauzeit und der Dosierung höherer Konzentrationen an Wirkstoffen im Medium wegen des zu erwartenden Zerfalls wärmesensibler Wirkstoffe bis zum aufgetauten Status zu lagern; ganz abgesehen davon, daß das Ausmaß des Zerfalls der wärmesensiblen Wirkstoffe lediglich ein grober Schätzwert ist, da die Auftauzeit wegen ihrer Abhängigkeit von der nicht ubiquitär vorzufindenden einheitlichen Zimmertemperatur Schwankungen unterliegen kann, so daß mit der Applikation höherer oder niedrigerer Wirkstoffmengen zu rechnen ist, ein Umstand, der zur Belastung des Patienten zu führen vermag.

Auch die weitere Möglichkeit, Spritzen durch Erwärmung mittels der Handfläche des Benutzers zu erwärmen, schließt eine Kontamination durch Mikroorganismen nicht aus. Zudem dauert das Auftauen in der Hand des Benutzers recht lange, wobei lediglich Injektionslösungen mit einem Volumen von 1 bis 2 ml innerhalb von 5 Minuten erwärmbar sind. Doch es ist gerade in Krankenhäusern bei der täglichen Routinearbeit, Notfallstationen oder bei Unfällen vor Ort dringend erforderlich und lebenswichtig, rasch Infusions- oder Injektionslösungen aufzutauen, welche bereits applikationsfertig und spritzfertig in Tiefkühlruhen gelagert werden.

Gerade das Auftauen in der Hand des Benutzers, welches lediglich für sehr geringe Flüssigkeitsvolumina geeignet ist, führt zu einem Mehraufwand an Zeit, da der Benutzer anderweitige Tätigkeiten, z. B. in Krankenhäusern, Intensivstationen, Notfallsituationen, nicht ausüben kann, was zu höheren Personalkosten führt.

Da das Auftauen von größeren Injektionslösungen, welche regelmäßig in Krankenhäusern verabreicht werden, durch Erwärmen mittels der Handfläche des Benutzers sehr zeitaufwendig ist, zumal die Wärmekapazität der Handoberfläche des Benutzers zu gering ist, und der Austausch oder das Vermischen von warmen und kalten Flüssigkeitsbereichen langwierig ist, sind die in auf den Kranken-, Intensiv- und Intensivstationen der Krankenhäuser und bei Notfallsituationen herkömmlichen Verfahren aufgrund langer Auftauzeit ungeeignet und es wird zudem wegen der mangelnden Sterilität dieser Verfahren dringend davon abgeraten.

Die WO-A-88 / 0 73 84, die zur Bildung Oberegriffs des Anspruchs 1 herangezogen worden ist, beschreibt einen Apparat zum Erhitzen und Mischen von Transfusions- und Infusionsflüssigkeiten mit einer Heizeinrichtung zur Aufnahme eines die gefrorene Flüssigkeit enthaltenden Behältnisses, welche mindestens eine Heizfläche und mindestens einen die Temperatur an der Außenwand des Behältnisses messenden Temperaturfühlers umfaßt. Die Heizeinrichtung (hier Heizgehäuse) wird in einer Ebene hin und zurück um eine Achse um etwa 220° mittels eines Hubzylinders schwenkbar bewegt. Die herkömmliche Lehre weist aber den Nachteil auf daß lediglich das Erwärmen der Transfusions- und Infusionsflüssigkeit durch die Schwenkung der Flüssigkeit mittels Wärmeleitung unterstützt wird, jedoch der Transport der Wärme durch strömende Flüssigkeit mit Verlagerung der Materie nicht stattfindet. Ausdrücklich macht die herkömmliche Lehre auf Seite 2 Absatz 2 darauf aufmerksam, daß diese auf das rasche und kontrollierte Erwärmen von Blut enthaltenden Behältnisses gerichtet ist. Die Erwärmung von Blut enthaltenden Flüssigkeiten hat besondere Erfordernisse, wie Vermeidung der Blutzellzerstörung, durch die dauerhafte Schwenkung während der Erwärmung und Verhinderung der Fragmentierung von hochmolekularen Molekülen, wie sie in Blut zu finden sind, durch schnelle Flüssigkeitsschüttlung zu berücksichtigen.

Auch die DE-A-30 47 784 betrifft eine Vorrichtung zum Erwärmen von eingefrorenen wäßrigen Suspensionen, wobei die Vorrichtung aus zwei wahlweise gegeneinander bewegbaren zur Aufnahme eines Gefriergut enthaltenden Kunststoffbeutels dienenden Heizplatten besteht, die von einem über einer feststehenden Grundplatte bewegbaren Haltelement getragen werden, das durch eine Schwenkvorrichtung rhythmisch ellipsenförmig schwenkbar ist Ebenso diese Lehre unterstützt das Auftauen des Gefriergutes, wobei unter Gefriergut lebende Zellsubstanz wie menschliche Blutkonserven zu verstehen ist; so daß ebenfalls die bereits oben genannten Erfordernisse bezüglich des Auftauens von lebenden Zellen in Flüssigkeiten zwar berücksichtigt werden, jedoch der Fachmann auch hier keine Hilfestellung erfährt, ein elektrisches Schnellauftaugerät bereitzustellen, bei welchem das Auftauen rasch erfolgt.

Aufgabe der vorliegenden Erfindung ist es, die o.g. Nachteile des Stands der Technik zu beseitigen. Zudem ist es wünschenswert, ein Schnellauftaugerät bereitzustellen, welches die Auftauzeit um ein Vielfaches verkürzt bei gleichzeitiger Schonung der Wirkstoffe und Einhaltung der dringenden lebensnotwendigen Sterilität. Zudem sollte es möglich sein, daß das Schnellauftaugerät eine einfache Mechanik aufweist, um nicht nur eine hohe Transportabilität zu gewährleisten sondern gleichzeitig eine geringe Wartung erforderlich zu machen. Weiterhin sollte das Schnellauftaugerät derart ausgebildet sein, daß eine rasche und hinreichende Reinigung und Sterilisierung seiner Bestandteile möglich ist, um häufig in Krankenhäusern auftretenden Hospitalismus zu vermeiden.

Die Aufgabe wird gelöst gemäß dem Kennzeichen des Hauptanspruchs. Die Unteransprüche betreffen bevorzugte Ausführungsformen und Weiterbildungen der Erfindung.

Die Erfindung gemäß Oberbegriff ist dadurch gekennzeichnet, daß der Linearantrieb ein hochdynamischer Lineardirektantrieb ist, der Bewegungsänderungen mit hoher Frequenz im Bereich von 20 bis 300 Hz ausführt, und der Lineardirektantrieb ein elektromechanischer Wandler zur Umwandlung von elektrischen Schwingungen in mechanische Schwingungen mit einer im wesentlichen in einer Ebene auslenkbaren Membran ist, welche über eine Schwingungsabgriffseinrichtung mit dem Übertragungsteller kraft-, reib- und / oder formschlüssig verbunden ist.

Hochdynamisch bedeutet im Sinne der Erfindung, daß der Lineardirektantrieb Bewegungsänderungen mit hoher Frequenz ausführt, die im Bereich von 20 bis 300 Hz, vorzugsweise 50 bis 200 Hz, noch mehr bevorzugt 50 bis 100 Hz, liegen. Ein Lineardirektantrieb ist ein Linearantrieb, welcher kraft-, form- und / oder reibschlüssig über z.B. ein Gestänge an den Übertragungsteller gekoppelt ist, um Bewegungen zu übertragen.

Durch das erfindungsgemäße Schnellauftaugerät erfolgt zudem das Auftauen einmal durch die Wärmeleitung, bei der die zu erwärmende kältere Flüssigkeit, die sich im Bereich des gefrorenen Flüssigkeitsanteils befindet, mit der wärmeren mit hoher Geschwindigkeit in innige Berührung gebracht wird, so daß die Atome und Molelüle des erwärmten Flüssigkeitsbereichs, die herkömmlicherweise im Mittel eine lebhafte ungeordnetere Bewegung als die kältere ausführen, nunmehr durch die durch das erfindungsgemäße elektrische Schnellauftaugerät hervorgerufenen hochfrequenten Schwingungen vorzugsweise in geordnetere Bewegungen in Richtung hin zu dem gefrorenen Flüssigkeitsbereich übergehen oder überlagert werden, und ihre Energie durch Stöße weitergeben.

Das erfindungsgemäße Schnellauftaugerät unterstützt hinzutretend bzw. beschleunigt die Wärmekonvektion innerhalb des Behältnisses, bei der die erwärmten Moleküle mit extrem hoher Bewegungsenergie in die Oberfläche der noch gefrorenen Wirkstoffe eindringen und somit den Wärmeaustausch beschleunigen und folglich die Auftauzeit drastisch verkürzen.

Durch die Verwendung des erfindungsgemäßen Schnellauftaugerät werden also in nahezu idealer Weise die die Wärmeübertragung hervorrufenden Parameter nicht nur unterstützt sondern in hinreichender Weise derart verstärkt, daß die Auftauzeiten der gefrorenen Flüssigkeit im Gegensatz zum Stand der Technik um ein Vielfaches, beispielsweise bei einer Spritze von 50 ml Volumen mit einer gefrorenen Flüssigkeit von gleichfalls 50 ml um ca. 20fach, verringert werden.

Als Behältnisse für das erfindungsgemäße Schnellauftaugerät eignen sich Spritzen, Ampullen, Infusionsflaschen, Infusionslösungsbeutel etc.. Die Größe, Länge und Gestalt der Spann-Heizeinrichtung, welche das Behältnis aufnimmt, ist entsprechend dem zu verwendenden Behältnis ausgebildet. Die Spann-Heizeinrichtung kann z.B hohlzylinderförmig für Spritzen, Infusionsflaschen etc. oder zur Aufnahme von z.B. Infusionslösungsbeuteln wannenförmig rechteckig ausgebildet sein. Hierbei weist die Spann-Heizeinrichtung eine Grundplatte als Basis und einen auf der Grundplatte koppelbaren Heizmantel auf welcher an die hohlzylinderförmige Wand des Spritzenkörpers anlegbar und z.B. auf der Außenseite mit einer thermisch isolierenden Deckschicht oder Coverschicht versehen ist. Die Grundplatte ist auf der Oberseite des Übertragungstellers mittels einer Schnellöseeinrichtung, wie Schraubverbindungen, koppelbar. Der Heizmantel kann zum Einführen und Aufnahme des Spritzenkörpers entsprechend dessen Form hohlzylinderförmig oder anderweitig entsprechend der zu benutzenden Behältnisse ausgestaltet sein.

Bei Ausbildung der Spann-Heizeinrichtung als hohlzylinderförmiger Heizmantel, bildet dieser einen z.B. den Spritzenkörper aufnehmenden Innenraum mit einer Mitte-Längsachse. Der Heizmantel ist hierbei im Querschnitt konzentrisch um die Mitte-Längsachse ausgebildet, so daß dieser der Form des Spritzenkörpers angepaßt und um diesen anlegbar ist. Der Heizmantel kann frontseitig -also auf der der Spritzenspitze zugewandten Seite- und rückseitig offen sein. Gleichfalls ist der Heizmantel an der Grundplatte koppelbar. Es ist von Vorzug, wenn der Heizmantel als Heiz- und Thermoisolationsmantel ausgestaltet ist. Auf der der Grundplatte gegenüberliegenden Seite findet sich ein vorzugsweise parallel zu der Mitte-Längsachse des Innenraums bzw. des Spritzenkörpers ausgerichteter Spalt, welcher die durch Wärme bedingte Materialausdehnung des Heizmantels und geringe Durchmesserunterschiede der z.B. Spritzenkörper ausgleicht Auch kann der Heizmantel als eine dem Spritzenkörper angepaßte vorgeformte Ausbildung sein, wobei der Innenraum des elastischen Heizmantels von oben durch Auseinanderspreizen der am Spalt gegenüberliegenden freien Enden zwecks Aufnahme des Spritzenkörpers zugänglich ist. Der Spritzenkörper kann hingegen auch rückseitig in den Innenraum des Heizmantels eingeführt werden.

Der Heizmantel kann zwei Heizseitenmänteln umfassen. Diese sind an der Grundplatte gelenkig gekoppelt und aus einer Öffnungsstellung zur Aufnahme des Behältnisses durch Schwenken um parallel zueinander ausgerichtete Drehachse in eine Schlußstellung überführbar. In der Schlußstellung liegen die Heizseitenmäntel mit der Heizfläche in innigem Kontakt auf der Wand des Spritzenkörpers. Inniger Kontakt bedeutet im Sinne der Erfindung, daß in der Schlußstellung ein möglichst geringer Luftspalt zwischen der Außenwand des Spritzenkörpers und der Heizfläche bzw. des Meßfühlers vorhanden ist.

Auf der dem Spritzenkörper abgewandten Seite des Heizmantels oder der Heizseitenmäntel können zumindest zwei Spannwandhälften um den Heizmantel oder die Heizseitenmäntel, welche im Querschnitt konzentrisch um die Mitte-Längsachse des Innenraums bzw. des Spritzenkörpers angeordnet sind, anlegbar sein. Zudem kann zwischen den beiden Drehgelenken ein Heizgrundmantel mit einer Heizfläche angeordnet sein, um eine allseitige Wärmeübertragung zu ermöglichen.

Die Spannwandhälften sind an der Grundplatte gekoppelt. Die freien oberen Enden der Spannwandhälften können mit Hilfe z.B. eines Schnellverriegelungsmechanismusses, wie eines Klinkenverschlusses, miteinander gekoppelt werden. Hierbei ist es von Vorteil, wenn die Spannwandhälften bei der Kopplung mittels der Klinkenverschlüsse mit einer Zugspannungskraft beaufschlagt werden, wobei der Heizmantel in z.B. vorgeformter und / oder elastischer Ausführung und / oder in Form von zwei Heizseitenmänteln innig an die Wand des Spritzenkörpers angedrückt wird, so daß man einen die Wärmeübertragung oder die Temperaturmessung behindernden Luftspalt möglichst vermeidet.

Weiterhin können Adapterhülsen aus herkömmlichen Materialien mit hoher Wärmeleitfähigkeit zwischen z.B. dem Spritzenkörper und dem Heizmantel angeordnet sein, welche geeignet sind, Wärme rasch zu übertragen und unterschiedliche Durchmesser von Spritzen, Flaschen etc. auszugleichen, so daß die Spann-Heizeinrichtung mit einem einheitlichen Durchmesser benutzt werden kann.

Die auf der Innenseite des Heizmantels, Heizseitenmäntel, Heizgrundmantel angeordnete Heizeinrichtung weist als Heizflächen herkömmliche Heizleiterwerkstoffe zur Umwandlung elektrischer Energie in Wärme mit hohem spezifischem Widerstand auf und ist dem Fachmann hinlänglich bekannt. Die Heizeinrichtung kann zudem an eine Überhitzungsschalteinrichtung angeschlossen sein. Die Heizfläche ist vorzugsweise ganzflächig auf der Innenseite des Heizmantels angebracht mit Ausnahme des oder der Bereiche, an denen wärmeisoliert von der Heizfläche Temperaturmeßfühler zu finden sind. Gerade die ganzflächige Ausgestaltung der Innenwände als Heizfläche ermöglicht ein rasches Auftauen gefrorener Flüssigkeiten, wie Injektions- oder Infusionslösungen, so daß die beim Sollwertgeber einstellbare Stellgröße von z.B. max. 35°C ausreichend ist, um die Auftauzeit im überragenden Maße im Gegensatz zum Stand der Technik zu verringern

Durch die einfache Ausbildung der von der Grundplatte abnehmbaren Spann-Heizeinrichtung ist gewährleistet, daß eine rasche und gründliche Reinigung und Sterilisierung ihrer Teile möglich ist, ohne daß für die Reinigungsflüssigkeiten oder Heißdampf schwer zugängliche, Mikroorganismen enthaltende Toträume vorhanden sind, ein Umstand, welcher gerade bei der Benutzung des erfindungsgemäßen Schnellauftaugerätes in Krankenhäusern, Notfallstationen oder Isolierstationen etc. zu beachten ist.

Der elektrische Lineardirektantrieb führt hochdynamische Bewegungen aus, die im wesentlichen in einer Dimension oder Ebene als Hin- und Herbewegungen erfolgen. Der elektrische Lineardirektantrieb ist ein elektromechanischer Wandler zur Umwandlung von elektrischen Schwingungen in mechanische mit einer schwingungsfähigen Membran. Hierzu eignet sich beispielsweise ein elektrischer Lineardirektantrieb, der nach Art eines elektrodynamischen Lautsprechers ausgebildet ist, der einen Schwingspulenträger, eine Membran und für die Stromzuführung der Schwingspule dienende flexible Verbindungsleitungen umfaßt, wobei die auf einer elektrischen Spule angeordnete Membran durch die Schwingungen der Spule innerhalb eines Magnetfeldes zu erzwungenen Schwingungen angeregt wird. Vorzugsweise kann ein nach Art eines Baß-Lautsprechers ausgestalteter elektrischer Lineardirektantrieb verwendet werden, bei dem die schwingende Membranfläche hinreichend groß und mit dem Übertragungsteller über eine Schwingungsabgriffseinrichtung verbunden ist. Im Sinne der Erfindung ist unter den im wesentlichen in einer Ebene erfolgten Schwingungen zu verstehen, daß bereits Auslenkungen in einer Ebene hinreichend sind, um den Wärmeaustausch zwischen dem erwärmten Flüssigkeitsbereich und dem gefrorenen zu gewährleisten. Zudem ist nicht auszuschließen, daß durch die Schwingungsabgriffseinrichtung möglicherweise Auslenkungen des Übertragungstellers in einer weiteren Ebene erfolgen oder daß bei der Verwendung von zwar groß dimensionierten Membranen, die jedoch nicht hinreichend steif ausgebildet sind, Partialschwingungen oder Verzerrungen auftreten können, die möglicherweise ebenfalls die in einer Ebene hin- und her erfolgenden Bewegungen überlagern, die jedoch durch das Führen des Übertragungstellers in Führungsschienen hinreichend vermieden werden.

Die Schwingungen oder Auslenkungen der Membran werden mittels einer Schwingungsabgriffseinrichtung abgegriffen und auf den Übertragungsteller und auf die auf dem Übertragungsteller gekoppelte Grundplatte übertragen. Die Membran ist mit dem Übertragungsteller kraft-, reib- und / oder formschlüssig verbunden. Bei der formschlüssigen Verbindung steht der Übertragungsteller über eine Gestänge oder ähnliche Verbindungsbauteile mit der Membran bzw. direkt mit dem elektrischen Lineardirektantrieb in Verbindung. Als Schwingungsabgriffseinrichtung kann in der einfachsten Art auch eine Flanschverbindung oder ein hohlzylinderförmiges Bauteil mittels Schraub-, Schweiß-, Nietverbindungen, Bolzenverbindungen und / oder Stiftverbindungen etc. zwischen der Membran und dem Übertragungsteller vorhanden sein.

Wenn die Schwingungsabgriffseinrichtung ein Gestänge umfaßt, ist das Gestänge in der einfachsten Ausgestaltung vorzugsweise mindestens eine Verbindungsstrebe, welche mit dem einem Ende mittig an der Membran und mit dem anderen Ende an dem Übertragungssteller gekoppelt ist. Die mechanische Schwingungskraft des z. B. Baß-Lautsprechers ist hinreichend, den Übertragungsteller des erfindungsgemäßen Schnellauftaugerätes in rasche Hin- und Herbewegungen zu führen, so daß die Wärmeströmung der erwärmten Flüssigkeit hin zu der kälteren stark unterstützt und der Austausch beider unterschiedlich temperierten Flüssigkeitsbereiche untereinander verstärkt wird. Die erwärmten Moleküle dringen mit großer Bewegungsenergie in die Oberfläche des gefrorenen Wirkstoffes ein und erwärmen dort wiederum partiell Moleküle, diese werden dann erst mitbewegt.

Aufgrund dieses Austausches erfolgt ebenfalls zwangsläufig der Wärmeaustausch zwischen dem kälteren schon aufgetauten Flüssigkeitsbereich mit dem noch gefrorenen Flüssigkeitsbereich abgesehen von dem weiter unten beschriebenen von dem erfindungsgemäßen Schnellauftaugerät hervorgerufenen Phänomen des 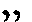 Torpedierens" der gefrorenen Flüssigkeit mit erwärmten Flüssigkeitsmolekülen.

Zudem ist zu beobachten, daß mittels des erfindungsgemäßen Schnellauftaugeräts gerade die Injektionslösungen in Spritzen oder Ampullen sehr schonend erwärmt werden, so daß im Gegensatz zum Stand der Technik eine chemische Modifikation, Denaturierung oder sonstige Veränderungen von Impfstoffen, Proteinen usw. nicht beobachtet wird. Das bedeutet, daß nunmehr fertig vorbereitete und formulierte, applizierbare Injektionsflüssigkeiten in Spritzen tiefgekühlt gelagert werden können und innerhalb einer sehr kurzen Auftauzeit dem Benutzer, wie Arzt, Krankenpflegepersonal, bedarfsgerecht gerade auch für den Routinealltag im Krankenhaus rasch zur Verfügung stehen.

Insbesondere zeigt sich desweiteren bei Notfallsituationen, bei denen größere fertig vorbereitete mit thermolabilen medizinischen Wirkstoffen versehene Injektionslösungen von 20 ml bis 100 ml zur Aufrechterhaltung körperlicher organischer Funktionen und bei Herz- Kreislautkomplikationen rasch zu applizieren sind, notfallgerecht dem Benutzer zur Verfügung stehen, ohne daß entweder ein langwährendes Auftauen dieser Injektionsflüssigkeiten oder ein zeitraubendes Vermischen von Ampullenflüssigkeiten und Wirkstoffen sowie deren Aufnahme in die Spritzen erforderlich ist.

Die Hin- und Herbewegungen des an den Schwingungsantrieb gekoppelten Übertragungstellers führt zu einer sehr raschen Verteilung der an der Innenwand des Spritzenkörpers erwärmten Lösung hin in Richtung zu der noch gefrorenen, vorwiegend zentrisch in der Spritze angeordneten Lösung. Das bedeutet, daß durch die rasche Hin- und Herbewegungen quasi die Flüssigkeitsmoleküle der gefrorenen Lösung mit Flüssigkeitsmolekülen der erwärmten Flüssigkeit 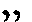 torpediert" werden und nicht nur der Wärmefluß von warm nach kalt unterstützt, sondern eine gleichmäßige und homogene Erwärmung der gefrorenen Injektions- oder Infusionslösung an ihrer gesamten noch gefrorenen Oberseite erreicht wird. Das 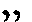 Torpedieren" mit warmen Flüssigkeitsmolekülen bewirkt auch ein Eindringen dieser in die gefrorene Flüssigkeit, so daß die gefrorene Flüssigkeit in ihrem Innern zusätzlich erwärmt wird und die Auftauzeit reduziert wird.

Hinzukommend ist zu beobachten, daß eine im Gegensatz zu den herkömmlichen Mikrowellenauftaugeräten auftretende Überhitzung oder zu starke übermäßige Erwärmung von Bereichen der Injektions- oder Infusionslösungen bei der Benutzung des erfindungsgemäßen Schnellauftaugerätes sich nicht feststellen läßt, da lediglich die an der Innenwand des Spritzenkörpers befindliche Flüssigkeit erwärmt wird und durch die Hin- und Herbewegung des Übertragungstellers eine miteinander kombinierte verstärkte Wärmeleitung und Wärmeströmung stattfinden.

Da in einer bevorzugten Ausführungsform die Membran eines als Schwingungsantrieb verwendeten elektrodynamischen Baß-Lautsprechers form- und kraftschlüssig mit dem Bewegungsteller des erfindungsgemäßen Schnellauftaugerätes verbunden ist, ist durch diesen Direktantrieb des Übertragungstellers gewährleistet, daß nicht nur wenig Schwingungsenergie aufzubringen ist, sondern zudem im Gegensatz zum Stand der Technik wenige bewegliche Teile zur Erzeugung der Schwingung des Übertragungstellers verwendet werden. Da die Anzahl der beweglichen Teile zur Erzeugung der Hin- und Rückbewegung des Bewegungstellers niedrig an Zahl und zudem einfach in ihrem Aufbau ist, zeigt das erfindungsgemäße Schnellauftaugerät eine niedrige Wartung und geringe Anfälligkeit gegenüber mechanischen Störungen mit hoher Haltbarkeit

Der auf der Innenseite der Heizwände der Spann-Heizeinrichtung angeordnete Temperaturmeßfühler kann die Temperatur der Wand des Spritzenkörpers messen und liefert ein Signal über Leitungen an die Regeleinrichtung. Die Regeleinrichtung ist eine dem Fachmann hinlänglich bekannte vorzugsweise unstetige oder stetige Regeleinrichtung. Die unstetige Regeleinrichtung kann einen Zweipunkt-Temperaturregler aufweisen, wobei zwei verschiedene Werte der Stellgröße, i.e. Temperatur von vorzugsweise 20 bis 35°C , noch mehr bevorzugt 25 bis 35°C, einstellbar sind. An dem Sollwertgeber können z. B. über einen Bedienungsteil wie Drehknopf verschiedene Werte der Stellgröße, i.e. der Temperatur z.B. 20 bis 35°C, eingegeben werden. Der Heizstrom als Stellgröße wird mittels der, vorzugsweisen elektronischen, Schalteinrichtung (Ein-, Ausschalter) ein- oder ausgeschaltet.

Der Temperaturregler kann über die Ansteuerschaltung den Heizstrom, z.B. bei Erreichen der voreingestellten höheren Temperatur, ab- und bei Erreichen der voreingestellten niedrigeren Temperatur wieder einschalten. Hierbei ist festzustellen, daß je mehr die Flüssigkeit aufgetaut ist, der Zeitraum, welcher zwischen dem Ausschalten und dem Einschalten der Heizeinrichtung liegt, größer wird. Entsprechend wird die Heizperiode, also das zeitweilige Einschalten der Heizeinrichtung, kürzer. Der Zweipunkt-Temperaturregler liefert bei Unterschreiten einer vorgegebener Temperatur ein Signal über die Ansteuerschaltung und die Schalteinrichtung, so daß die Heizeinrichtung nur eingeschaltet wird, solange der Temperaturregler das Signal liefert. Die Zeiträume von dem Ausschalten bis zum Einschalten der Heizeinrichtung mittels der Schalteinrichtung bzw. deren Anzahl (Nichtheizperiode) können als Maß für den aufgetauten Zustand der Flüssigkeit ausgewertet werden, wobei bei Überschreiten einer vorgegebenen Temperatur der Temperaturregler kein Signal mehr liefert. Die Abnahme des Zeitraums ist als Maß für den aufgetauten Zustand der Flüssigkeit auswertbar und auf eine Anzeige, welche mit dem Temperaturregler verbunden ist, anzeigbar.

Die Heizwände der Spann-Heizeinrichtung können mit Textilgewebe mit auf der Innenseite angeordneten eingewebten Heizdrähten versehen sein, wobei der Temperaturmeßfühler wärmeisoliert beabstandet von der Heizeirrichtung bzw. deren Heizflächen auf der Wand des Spritzenkörpers anlegbar ist. Über ein elektrisch steuerungstechnisch an die Regeleinrichtung angeschlossenes Bedienfeld kann die Größe der Spritze bzw. das Volumen der aufzutauenden Flüssigkeit eingegeben werden, so daß auch in diesem Falle das erfindungsgemäße Schnellauftaugerät aufgrund der Zunahme der Nichtheizperiode bzw. deren Anzahl den Status der verwendeten Flüssigkeit in der Spritze anzuzeigen vermag.

Die Regeleinrichtung kann auch vom Dreipunkt-Typ sein, wobei drei verschiedene Werte der Stellgrößen, hier Temperatur, verwendet werden. Bei der Dreipunkt-Regeleinrichtung finden zwei Heizeinrichtungen Verwendung, wobei die eine Heizeinrichtung von großer Heizleistung und die andere von kleiner Heizleistung ist. Hierbei ist es möglich, daß die größere Heizeinrichtung in der Regel dauernd eingeschaltet und die Grundlast bildet und die kleinere je nach Wärmebedarf zugeschaltet werden kann und die Zusatzlast bildet. Vorteilhaft ist hierbei, daß die Änderung der gesteuerten Temperaturleistung und die Ein- und Ausschaltzeit bzw. die Schwankungen um eine gewünschte Temperatur geringer sind.

Durch die gleichmäßige Erwärmung der Flüssigkeit in dem der Wand des Spritzenkörpers zugewandten Bereich und die überaus hohe Verstärkung der Wärmekonvektion durch Transport von warm nach kalt wird nicht nur die Flüssigkeit schonend bei einer Temperatur erwärmt, welche nicht zu einer Schädigung der medizinisch wirksamen Arzneimittel in der Injektionsflüssigkeit führt, sondern es ist auch erstmals möglich, nur noch soviel an medizinisch wirksamer Substanz in die Injektionsflüssigkeit zu geben, wie tatsächlich für den Organismus des Patienten erforderlich ist, denn im Gegensatz zum Stand der Technik ist bei Einsatz des erfindungsgemäßen Schnellauftaugerätes nicht mit einem Zerfall oder einer Einschränkung der therapeutisch wirksamen Menge des Arzneimittels zu rechnen. Das bedeutet aber auch, daß abgesehen von der nunmehr genau dosierbaren therapeutisch notwendigen Applikationsmöglichkeit ein überflüssiger Mehrverbrauch an Arzneimitteln nicht mehr stattfindet und zudem der Mehraufwand an Personal, welcher herkömmlicherweise zum kontrollierten Auftauen erforderlich wäre, nicht entsteht; Umstände also, die für den Fachmann als unerwartet anzusehen sind, da Vorteile, wie rasches Auftauen, mangelnde Schädigung therapeutisch wirksamer Substanzen in der aufgetauten Flüssigkeit sowie Fehlen von Kontamination durch Mikroorganismen und damit hinreichende Gewähr von Sterilität während des Auftauvorgangs, in deren Kombination erstmals mit Hilfe des erfindungsgemäßen Schnellauftaugeräts in einer nahezu idealen Weise gerade für den Einsatz in Krankenhäuser im Alltagsbetrieb und in Notfallsituationen erreichbar sind.

Die Ansteuerschaltung für den Temperaturregler, der elektrische Lineardirektantrieb, die Heizeinrichtung etc., die Schalteinrichtungen, der Temperaturmeßfühler können mit der Anzeige und / oder dem Bedienungsfeld elektrisch-steuerungstechnisch auf herkömmliche Weise verbunden sein. Hierbei ist es möglich, über eine dem Fachmann hinlänglich bekannte Auswerteeinrichtung die von der Ansteuerschaltung gesendeten Signale auf der Anzeige abzubilden und, falls erforderlich, die Zunahme des Zeitraumes von dem Ausschalten bis zum Einschalten der Heizeinrichtung anzuzeigen, als Maß für den aufgetauten Zustand der Flüssigkeit auszuwerten und dieses auf der Anzeige darzustellen.

### Ausführungsbeispiele

Weitere Einzelheiten, Aspekte und Vorteile der Erfindung ergeben sich aus der nachfolgenden Beschreibung unter Bezugnahme auf die Zeichnungen. Es zeigen aufgrund einer zeichnerische Vereinfachung in schematischer stark vergrößerter Weise, ohne Anspruch auf maßstabsgetreue Wiedergabe
- **Fig. 1**: den Längsschnitt einer Spann-Heizeinrichtung des erfindungsgemäßen Schnellauftaugerätes
- **Fig. 2**: die Draufsicht auf die Spann-Heizeinrichtung des erfindungsgemäßen Schnellauftaugerätes
- **Fig. 3**: die Ansicht des Schnitts A-B und des Schnitts C-D nach Fig. 1
- **Fig. 4**: die perspektivische Schrägansicht auf das erfindungsgemäße Schnellauftaugerät
- **Fig. 5**: den Längsschnitt und die Draufsicht einer Spann-Heizeinrichtung des erfindungsgemäßen Schnellauftaugerätes mit einer an den Linearantrieb gekoppelten Schwingungsabgriffseinrichtung, welche mit dem Übertragungsteller verbunden ist
- **Fig. 6**: den Schnitt mit der Ansicht des Schnitts A-B nach Fig. 5

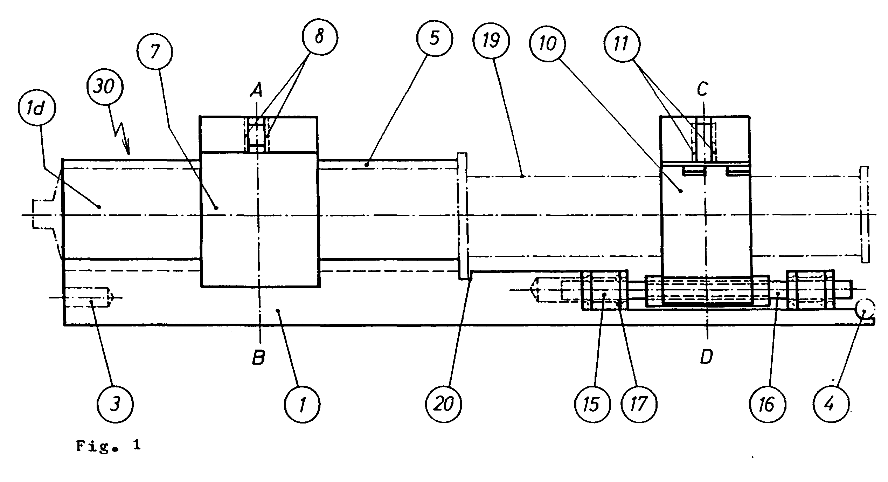

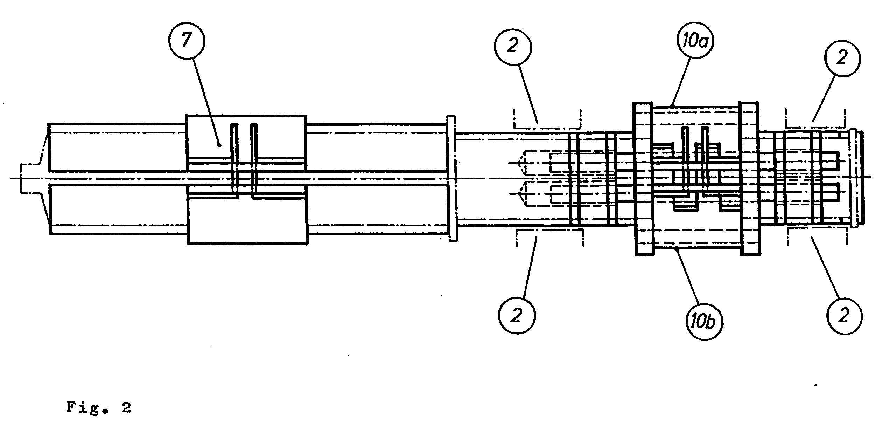

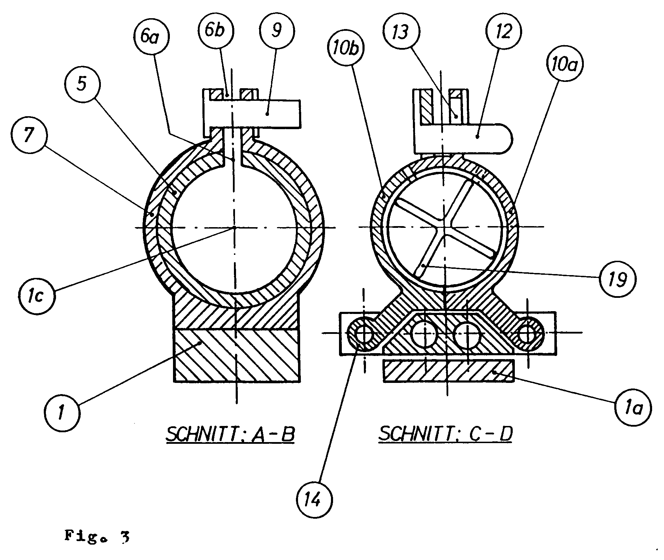

Auf dem Übertragungsteller 1a ist eine Grundplatte 1, welche über Schnellöseseinrichtungen, hier Spannverschluß 3, 4 (oder auch über Verbindungsschrauben oder Klammern), mit dem Übertragungsteller 1a verbunden ist, vorhanden. Die Spann-Heizeinrichtung 30 ist hohlzylinderförmig ausgebildet, welcher einen Heiz- und Thermoisolationsmantel 5 mit einem oberseitig angeordneten Schlitz oder Spalt 6a zum Ausgleich von Maßabweichungen bei der Erwärmung und von unterschiedlichen Spritzenkörperdurchmessern umfaßt. Die an den Spalt 6a gegenüberliegenden freien Enden des Heiz- und Thermoisolationsmantels 5 können in etwa tangential auseinander gespreizt werden (Öffungsstellung), um die Spritze mit Spritzenkörper 1d längs einzulegen. Auf der dem Innenraum zugewandten Seite des Heiz- und Thermoisolationsmantels 5 befinden sich die Heizeinrichtungen mit Heizflächen, welche nicht abgebildet sind. Die Größe und damit der Durchmesser des Heiz- und Thermoisolationsmantels 5 entspricht in etwa der üblichen Norm des Außendurchmessers des Spritzenkörpers 1d der jeweiligen Spritzengröße von 50 ml Volumen. Konzentrisch um den Heiz- und Thermoisolationsmantel 5 finden sich zwei Spannwandhälften 7. Diese sind an der Basis mit der Grundplatte 1 fest verbunden. Oberseitig gegenüber der Grundplatte 1 sind an den freien Enden der Spannwandhälften 7 unter Bildung eines zweiten Spaltes 6b Spannklinkenverschlüsse 9 angebracht (Fig. 1 -3).

In der Öffnungsstellung der Klinkenverschlüsse 9 kann die Spritze mit dem Spritzenkörper 1d in den von dem Heiz- und Thermoisolationsmantel 5 gebildeten Innenraum eingeführt werden. In der Schlußstellung der Klinkenverschlüsse 8, 9 (Klinkenhebel 9, Klinkenverschluß mit Verzahnung 8, Öffnen durch Zusammendrücken der beiden Klinkenhebel 9) werden die z.B. elastischen, wie zugelastischen, Spannwandhälften 7 mit einer Zugspannungskraft derartig beaufschlagt, daß der Heiz- und Thermoisolationsmantel 5 an dem Spritzenkörper 1d innig anliegt, so daß kein wesentlicher die Wärmeübertragung und die -messung hindernder Luftspalt vorhanden ist.

Um bei den Hin- und Herbewegungen während des Auftauvorgangs ein mögliches Verschieben des Kolbens 19 der Spritze z.B. in Längsrichtung des Spritzenkörpers 1d zu verhindern, wird der Spritzenkolben 19 gleichfalls mittels Spannhälften 10 (rechte Spannhälfte 10a, linke Spannhälfte 10b) gesichert, welche mit Hilfe von Spannklinkenverschlüssen 11 z.B. mit Verzahnung versehen sind; das Öffnen erfolgt durch Zusammendrücken der beiden Klinkenhebel (Klinkenhebel 12, Klinkenhebeldurchführung 13), die ebenso mit einer Spannkraft beaufschlagbar in Schlußstellung einstellbar sind. Die Spannhälften 10 können über Drehgelenke 14 an der Grundplatte 1 schwenkbar (nicht gezeigt) gekoppelt sein. Die Drehgelenke 14 können auf der Oberseite der Grundplatte 1 angeordnet und auch in Längsrichtung parallel zu der Mitte-Längsachse 1c des Spritzenkörpers 1d auf der Grundplatte 1 verschiebbar einstellbar sein. Die Verschiebbarkeit der Drehgelenke 14 wird durch Gleitlager 15 ermöglicht, wobei Führungsstangen 16 durch Gleitlager 15 parallel zu der Mitte-Längsachse 1c des Spritzenkörpers 1d verschiebbar sind. Die Drehgelenke 14 sind an die Führungsstangen 16 gekoppelt sowie die Führungsstangen 16 in den Gleitlagern 15 mittels Einstellschrauben an der Grundplatte 1 einstellbar. Gummiringe 17 dienen als Puffer zum Dämpfen der Gleitlagerung. Einstellbare Seitenführungen 2 auf dem Übertragungsteller 1 a dienen zum leichten Einlegen der Spann-Heizeinrichtung 30. In eine Nut 20 in der Grundplatte 1 greift der nach außen auskragende Rand des Spritzenkörpers 1d, so daß der Spritzenkörper 1d in Längsrichtung festgehalten wird.

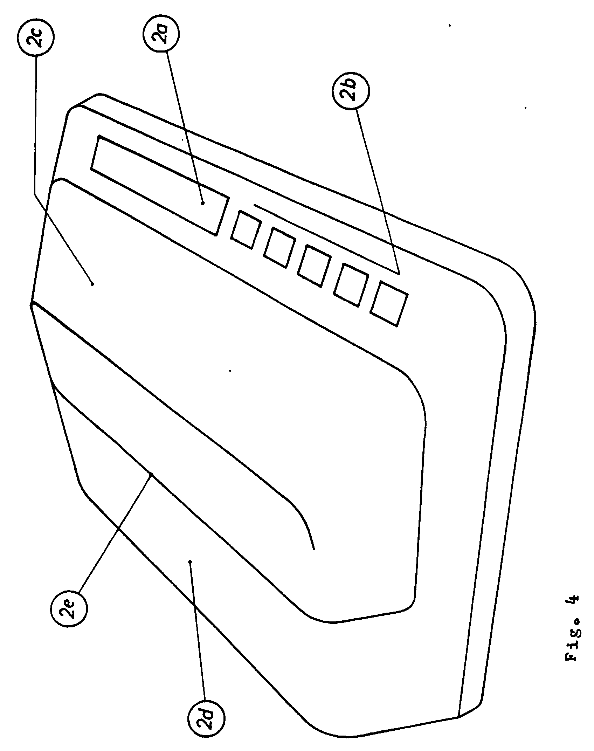

Auf der Innenseite des Heiz- und Thermoisolationsmantels 5 ist gleichfalls von der Heizeinrichtung wärmeisoliert beabstandet ein Temperaturmeßfühler (nicht gezeigt) angebracht, welcher ein Signal über Leitungen an eine elektrische ZweipunktRegeleinrichtung liefert, und dessen Ausgang mit dem Temperaturregler der elektrischen Zweipunkt- Regeleinrichtung verbunden ist. Die Ansteuerschaltung der elektrischen Zweipunkt-Regeleinrichtung liefert an ihrem Ausgang ein im wesentlichen dem Vergleich der durch den Temperaturmeßfühler erfaßten Temperatur des Spritzenkörpers mit dem vorgegebenen über das Bedienungsteil 2b eingegebenen Sollwert entsprechendes Signal, das über die Schalteinrichtung (Ein-Ausschaltung) die Verbindung zu der Heizeinrichtung herstellt oder unterbricht. Gleichfalls wird ein Signal von der Ansteuereinrichtung an die Schalteinrichtung (Ein-Ausschaltung) des elektrischen Schwingungsantriebes geliefert. Eine Stromversorgung versorgt die elektrische Zweipunkt-Regeleinrichtung mit der Ansteuerschaltung und die u.a. mit dem Temperaturregler verbundene Anzeige 2a . Die Ansteuerschaltung ist eingangsseitig mit dem Temperaturmeßfühler und dem Sollwertgeber verbunden, der über das Bedienungsfeld 2b über Tasten oder Drehknopf einstellbar ist. Weiter versorgt u.a. die Stromversorgung eine mit dem Ausgang der Ansteuerschaltung verbundene Schalteinrichtung für die Heizeinrichtung und den elektrischen Schwingungsantrieb sowie die ebenfalls mit dem Ausgang der Ansteuerschaltung verbundene Anzeige 2a, die beispielsweise durch Leuchtdioden oder durch LCD gebildet ist (Fig. 4).

Als elektrischer Schwingungsantrieb findet ein dem Prinzip des Baß-Lautsprechers ausgestalteter Antrieb Verwendung, wobei der Übertragungsteller 1a über ein Gestänge mit der Membran form- und kraftschlüssig verbunden ist.

Bei Vergleichsversuchen zeigt sich, daß bei einer geregelten Temperatur an der Spritzenoberfläche zwischen 25 bis 35°C bei Verwendung einer 50 ml-Spritze mit 50 ml gefrorener Flüssigkeit die Zeit bis zum aufgetauten Zustand der Flüssigkeit um den Faktor 20 verringert wird, im Vergleich zum Stand der Technik, wobei als Kontrolle eine Spritze identischer Größe und Flüssigkeitsvolumen bei normaler Konvektion aufgetaut wird. Weiterhin ist zu beachten, daß in der Kontrolle zusätzlich Zeit von dem Benutzer zum Einstellen der Wassertemperatur auf handwarm benötigt wurde -was nicht in dem Vergleichsergebnis berücksichtigt wird- sowie das Empfinden des Benutzers, handwarmes Wasser einzustellen, individuell unterschiedlich ist, so daß zudem im Stand der Technik nicht gewährleistet wird, Injektionsflüssigkeiten immer bei gleichbleibender Temperatur von verschiedenen Benutzern aufzutauen.

Die auf der Oberseite des Übertragungstellers 1a gekoppelte Spann-Heizeinrichtung ist, falls erforderlich, mittels eines Sicherheitsverschlusses 2c verdeckt, welcher über rückseitige Drehgelenke 2e an dem Gehäuse 2d des erfindungsgemäßen Schnellauftaugeräts gekoppelt ist.

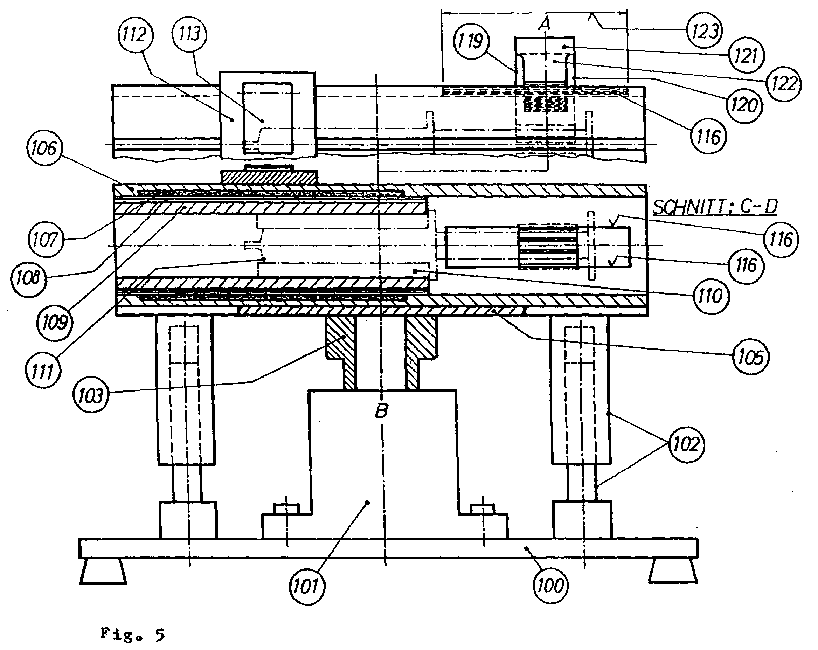

In Fig. 5 ist als Schwingungsabgriffseinrichtung ein hohlzylinderförmiges Bauteil 103 an eine Membran eines Linearantriebes form- und kraftschlüssig gekoppelt. Der Linearantrieb wird wegen der form- und kraftschlüssigen mechanischen Kopplung auch Lineardirektantrieb genannt. Der Linearantrieb arbeitet nach dem Prinzip eines elektrodynamischen Lautsprechers mit einer im wesentlichen in Hin- und Herbewegungen in Richtung zum Beispiel der X- Achse in Schwingungen versetzten Membran. Da Linearantriebe hinlänglich bekannt sind, ist ein solcher nicht abgebildet. Der Linearantrieb befindet sich in einem hohlzylinderförmigen Gehäuse 101 mit oberseitiger Öffnung. Aus der Öffnung wird das Bauteil 103 geführt und ist an den Übertragungsteller 105 form- und kraftschlüssig gekoppelt. Die Schwingungen der Membran werden über das Bauteil 103 auf den Übertragungsteller 105 übertragen. Der Übertragungsteller 105 weist an den Seiten zumindest zwei auf einer Grundplatte 100 befindliche Führungssäulen oder -schienen 102 auf um die Hin- und Herbewegungen vorwiegend in X-Richtung zu ermöglichen. Die Spann-Heizeinrichtung 30 umfaßt eine thermische Isolationsschicht 107, welche eine Heizwand 108 mit Heizeinrichtung und zum Innenraum liegenden Heizflächen umgibt.

Metallhülsen 109 mit hinreichender Wärmeleitfähigkeit sind auf der dem Innenraum zugewandten Seite der Heizwand 108 angeordnet. Adapterhülsen 110 ebenfalls mit großer Wärmeleitfähigkeit können verwendet werden, falls der Durchmesser des im Innenraum aufgenommenen Spritzenkörpers 111 so gering ist, daß die Metallhülse 109 nicht hinreichend auf dem Wand des Spritzenkörpers 111 anliegt, sondern von diesem beabstandet ist. Jegliches Material mit großer thermischer Leitfähigkeit eignet sich für die Metallhülsen 109 und Adapterhülsen 110 und sind dem Fachmann hinlänglich bekannt. Die Bezugszeichen 112 (Spannvorrichtung) und 113 (Spann-/Verschlußhebel) stehen für bereits oben beschriebenen Spannklinkenverschlüsse bzw. deren Teile. Das Spanngehäuse 106 entspricht im wesentlichen den oben genannten Spannwandhälften 7.

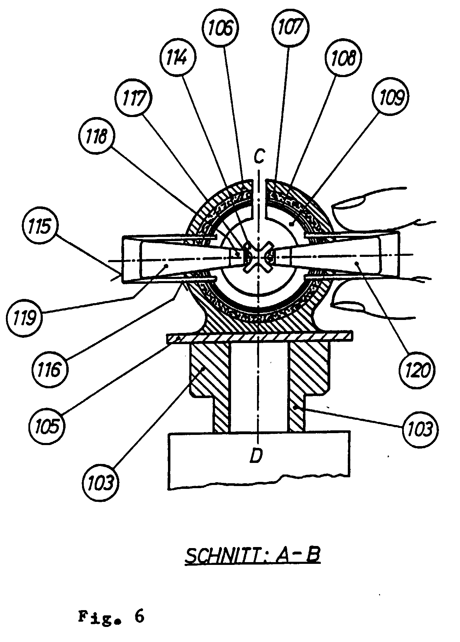

Die Kolbenstange oder Kolben 114 (Fig. 6) der Spritze wird von einer Klemmeinrichtung 115 geführt. Die Klemmeinrichtung dient zur vibrationsfreien Lagerung der Kolbenstange 114 der Spritze in vertikaler Richtung. Die Klemmeinrichtung 115 macht gleichzeitig die translatorischen Bewegungen mit, die durch die Volumenverringerung infolge Wärme -vom gefrorenen Zustand der Flüssigkeit mit Wirkstoff auf bis zu 4°C- und die anschließende Volumenvergrößerung infolge weiterer Erwärmung -der Flüssigkeit mit Wirkstoff von 4°C auf 35°C- erfolgen. Die herkömmliche Klemmeinrichtung 115 umfaßt zwei seitlich angeordnete Federbleche 119, 120. Bei der Wärmeausdehnung biegen sich beispielsweise die Federbleche 119, 120 in Längsrichtung leicht, in vertikaler Richtung verbleiben sie steif. Die Klemmeinrichtung 115 weist ober- und unterseitig Rastfedern 122 auf welche unterseitig und oberseitig mit einer Verzahnung 116 arretierbar sind. Am freien Ende der Federbleche 119, 120 angeordnete Gummianschläge 117 ermöglichen ein hinreichendes Anschmiegen und Führen der Federbleche 119, 120 an der Kolbenstange 114. In Abhängigkeit von der Größe der Kolbenstange 114 kann die Klemmeinrichtung 115 in einem Verstellbereich 123 einstellbar sein. Obere und untere Metallstücke 118, 121 dienen zur Befestigung von Federblechen 119, 120. Das obere Metallstück 121 dient zusätzlich zur Befestigung der Rastfedern 122.

Weiterhin zeigt sich, daß durch heftiges Schütteln der Spritzen unter fließendem warmem Wasser ein Bewegen des Spritzkolbens 19 parallel zu der Mitte-Längsachse 1c des Spritzenkörpers nicht auszuschließen ist, was entweder zu einem Herausdrükken der Injektionsflüssigkeit oder zu einem Aspirieren von Mikroorganismen entweder in dem Spalt zwischen dem Spritzkolben 19 und Innenwand des Spritzenkörpers 1d oder über die Spritzenöffnung erfolgt. Demgegenüber zeichnet sich das erfindungsgemäße Schnellauftaugerät nicht nur durch eine sehr kurze Auftauzeit aus sondern gewährleistet in hinreichendem Maße, ein Auftauen unter Beachtung von Sterilität bei dem Erwärmen der gefrorenen Flüssigkeit auf eine Temperatur, die der gewünschten, vorzugsweise der Körpertemperatur entspricht. Auch dieser Umstand der Einstellbarkeit der aufzutauenden Flüssigkeit auf Körpertemperatur ist von dem medizinischen Standpunkt erforderlich, da bekannterweise Patienten das Injizieren von nicht körpertemperaturgerechter Flüssigkeiten zumindest als unangenehm, wenn nicht gar als störend, empfinden.

Somit stellt das erfindungsgemäße Schnellauftaugerät eine rasch arbeitende, schonende und Personal einsparende Vorrichtung dar, welches aufgrund seiner einfachen Mechanik zudem schnell zu reinigen oder zu desinfizieren, wartungsarm sowie wenig reparaturanfällig ist.

## Patentansprüche

1. Elektrisches Schnellauftaugerät zum raschen Auftauen einer gefrorenen Flüssigkeit, beispielsweise einer Injektions- oder Infusionslösung, mit einem Übertragungsteller (1a, 105),
einer Spann-Heizeinrichtung (30) zur Aufnahme eines die gefrorene Flüssigkeit enthaltenden Behältnisses, insbesondere von einer Spritze oder einer Ampulle,
welche eine elektrisch beheizbare Heizeinrichtung mit mindestens einer Heizfläche und mindestens einen die Temperatur an der Außenwand des Behältnisses messenden Temperaturmeßfühler umfaßt,
sowie einer elektrischen Regeleinrichtung mit einem Temperaturregler mit Ansteuerschaltung, wobei der Temperaturmeßfühler und ein Sollwertgeber ausgangsseitig mit der Ansteuerschaltung verbunden sind, die Ansteuerschaltung ausgangsseitig mit einer Schalteinrichtung zur Steuerung der Heizeinrichtung verbunden ist, wobei die Schalteinrichtung in Abhängigkeit von Signalen des an dem Eingang der Ansteuerschaltung angeschlossenen Temperaturmeßfühlers und Sollwertgebers die Heizeinrichtung steuert,
der Übertragungsteller (1a, 105) an einen elektrischen Linearantrieb gekoppelt und im wesentlichen in einer Ebene bewegbar ist und auf dem Übertragungsteller (1a, 105) die Spann-Heizeinrichtung (30) koppelbar ist, **dadurch gekennzeichnet,** daß
der Linearantrieb ein hochdynamischer Lineardirektantrieb ist, der Bewegungsänderungen mit hoher Frequenz im Bereich von 20 - 300 Hz ausführt, und der Lineardirektantrieb ein elektromechanischer Wandler zur Umwandlung von elektrischen Schwingungen in mechanische Schwingungen mit einer im wesentlichen in einer Ebene auslenkbaren Membran ist, welche über eine Schwingungsabgriffseinrichtung mit dem Übertragungsteller (1a, 105) kraft-, reib- und / oder formschlüssig verbunden ist.

2. Elektrisches Schnellauftaugerät zum raschen Auftauen von gefrorenen Flüssigkeit nach Anspruch 1, **dadurch gekennzeichnet,** daß die Schwingungsabgriffseinrichtung eine Flanschverbindung ist.

3. Elektrisches Schnellauftaugerät zum raschen Auftauen von gefrorenen Flüssigkeit nach Anspruch 1, **dadurch gekennzeichnet,** daß die Schwingungsabgriffseinrichtung ein Gestänge ist, bei welchem mindestens eine Verbindungsstrebe mit dem einem Ende mittig an der Membran und mit dem anderen Ende an dem Übertragungsteller (1a, 105) gekoppelt ist.

4. Elektrisches Schnellauftaugerät zum raschen Auftauen von gefrorenen Flüssigkeit nach einem der Ansprüche 1 bis 3, **dadurch gekennzeichnet,** daß der elektrische Lineardirektantrieb ein Baßlautsprecher ist.

5. Elektrisches Schnellauftaugerät zum raschen Auftauen von gefrorenen Flüssigkeit nach einem der Ansprüche 1 bis 4, **dadurch gekennzeichnet,** daß die Spann-Heizeinrichtung (30) als Basis eine Grundplatte (1) aufweist, welche mit dem Übertragungsteller (1a) über Schnellöseeinrichtungen verbunden ist.

6. Elektrisches Schnellauftaugerät zum raschen Auftauen von gefrorenen Flüssigkeit nach einem der Ansprüche 1 bis 5, **dadurch gekennzeichnet,** daß der Übertragungsteller (1a, 105) an den Seiten mindestens zwei auf einer Grundplatte (100) angeordnete Führungsschienen (102) zur Ermöglichung der Hin- und Herbewegungen aufweist.

7. Elektrisches Schnellauftaugerät zum raschen Auftauen von gefrorenen Flüssigkeit nach einem der Ansprüche 1 bis 6, **dadurch gekennzeichnet,** daß der Temperaturmeßfühler mindestens an einer Wand des Behältnisses im innigen Kontakt anlegbar ist.

8. Elektrisches Schnellauftaugerät zum raschen Auftauen von gefrorenen Flüssigkeit nach einem der Ansprüche 1 bis 7, **dadurch gekennzeichnet,** daß die Spann-Heizeinrichtung (30) einen hohlzylinderförmigen Heizmantel umfaßt.

## Claims

1. Electrical high-speed defrosting appliance for rapid defrosting of a frozen liquid, for example an injection or infusion solution, including a transmission disk (1a, 105),
a clamp heating device (30) for holding a receptacle containing the frozen liquid, especially a syringe or an ampoule,
which comprises an electrically heatable heating device having at least one effective heating surface and at least one temperature sensor measuring the temperature on the outer wall of the receptacle,
as well as an electrical control system having a temperature control device with a drive circuit, the temperature sensor and a setpoint generator being connected with the drive circuit on the output side, the drive circuit being connected with circuitry on the output side for controlling the heating device, the circuitry controlling the heating device as a response to signals from the temperature sensor and the setpoint generator attached at the drive circuit input,
the transmission disk (1a, 105) being coupled with an electrical linear drive and being essentially movable in one plane, and the clamp heating device (30) being adapted to be coupled to the transmission plate (1a, 105), characterized in that the linear drive is a highly dynamic linear direct drive which carries out changes of movement at a high frequency in a range of from 20 to 300 Hz, and the linear direct drive is an electromechanical convertor for transforming electrical oscillations into mechanical vibrations by means of a membrane which is deflectable essentially in one plane and which is connected with the transmission disk (1a, 105) via a vibration pick-up device in a non-positive, frictional and/or positive manner.

2. Electrical high-speed defrosting appliance for rapid defrosting of frozen liquid as claimed in Claim 1, characterized in that the vibration pick-up device is a flanged joint.

3. Electrical high-speed defrosting appliance for rapid defrosting of frozen liquid as claimed in Claim 1, characterized in that the vibration pick-up device is a linkage in which at least one connecting strut is coupled with one end centrally to the membrane and with the other end to the transmission plate (1a, 105).

4. Electrical high-speed defrosting appliance for rapid defrosting of frozen liquid as claimed in any one of Claims 1 to 3, characterized in that the electrical linear direct drive is a bass speaker.

5. Electrical high-speed defrosting appliance for rapid defrosting of frozen liquid as claimed in any one of Claims 1 to 4, characterized in that the clamp heating device (30) has a base plate as its base (1) which is connected with the transmission disk (1a) via quick release means.

6. Electrical high-speed defrosting appliance for rapid defrosting of frozen liquid as claimed in any one of Claims 1 to 5, characterized in that the transmission plate (1a, 105) has on its sides at least two guide rails (102) arranged on a base plate (100) to allow oscillating movements.

7. Electrical high-speed defrosting appliance for rapid defrosting of frozen liquid as claimed in any one of Claims 1 to 6, characterized in that the temperature sensor can be placed in intimate contact with at least one wall of the receptacle.

8. Electrical high-speed defrosting appliance for rapid defrosting of frozen liquid as claimed in any one of Claims 1 to 7, characterized in that the clamp heating device (30) comprises a hollow cylindrical heating jacket.

## Revendications

1. Appareil électrique de décongélation rapide pour décongeler rapidement un liquide congelé, par exemple une solution d'injection ou de perfusion, comportant un plateau de transmission (1a, 105),
un dispositif de chauffage à serrage (30) servant à recevoir un récipient contenant le liquide congelé, notamment une seringue ou une ampoule_{,}
qui comprend un dispositif de chauffage pouvant être chauffé électriquement et comportant au moins une surface de chauffage et au moins un capteur de mesure de température mesurant la température sur la paroi extérieure du récipient,
ainsi qu'un dispositif de régulation électrique comportant un régulateur de température possédant un circuit de commande, le capteur de mesure de température et un capteur de valeur de consigne étant reliés, côté sortie, au circuit de commande, le circuit de commande étant relié, du côté sortie, à un dispositif de commutation pour la commande du dispositif de chauffage, et le dispositif de commutation commandant le dispositif de chauffage en fonction de signaux du capteur de mesure de température raccordé à l'entrée du circuit de commande et du générateur de valeur de consigne,
le plateau de transmission (1a, 105) étant couplé à un dispositif électrique d'entraînement linéaire et pouvant être déplacé essentiellement dans un plan, et le dispositif de chauffage à serrage (30) pouvant être couplé au plateau de transmission (1a, 105),
caractérisé en ce que
le dispositif d'entraînement linéaire est un dispositif d'entraînement linéaire direct à dynamique élevée, qui exécute des modifications de déplacement avec une fréquence élevée dans la gamme de 20-300 Hz, et le dispositif d'entraînement linéaire direct est un convertisseur électromécanique servant à convertir des oscillations électriques en oscillations mécaniques avec une membrane pouvant être déviée essentiellement dans un plan et qui est reliée selon une liaison de force, une liaison à friction et/ou une liaison par formes complémentaires, au plateau de transmission (1a, 105) par l'intermédiaire d'un dispositif de captage d'oscillations.

2. Appareil électrique de décongélation rapide pour décongeler rapidement un liquide congelé selon la revendication 1, caractérisé en ce que le dispositif de captage d'oscillations est une liaison à bride.

3. Appareil électrique de décongélation rapide pour décongeler rapidement un liquide congelé selon la revendication 1, caractérisé en ce que le dispositif de captage d'oscillations est une tringlerie, dans laquelle au moins une entretoise de liaison est couplée par une extrémité au centre de la membrane et par l'autre extrémité au niveau du plateau de transmission (1a, 105).

4. Appareil électrique de décongélation rapide pour décongeler rapidement un liquide congelé selon l'une des revendications 1 à 3, caractérisé en ce que le dispositif d'entraînement électrique linéaire direct est un haut-parleur pour les graves.

5. Appareil électrique de décongélation rapide pour décongeler rapidement un liquide congelé selon l'une des revendications 1 à 4, caractérisé en ce que le dispositif de chauffage à serrage (30) comporte comme base une plaque de base (1), qui est reliée au plateau de transmission (1a) par l'intermédiaire d'un dispositif de détachement rapide.

6. Appareil électrique de décongélation rapide pour décongeler rapidement un liquide congelé selon l'une des revendications 1 à 5, caractérisé en ce que le plateau de transmission (1a, 105) comporte, sur les côtés, au moins deux rails de guidage (102) disposés sur une plaque de base (100) et permettant des déplacements en va-et-vient.

7. Appareil électrique de décongélation rapide pour décongeler rapidement un liquide congelé selon l'une des revendications 1 à 6, caractérisé en ce que le capteur de mesure de température peut être placé en contact intime au moins contre une paroi du récipient.

8. Appareil électrique de décongélation rapide pour décongeler rapidement un liquide congelé selon l'une des revendications 1 à 7, caractérisé en ce que le dispositif de chauffage avec serrage (30) comporte une enveloppe chauffante en forme de cylindre creux.
